# EUROPEAN PATENT SPECIFICATION

(11) **EP 4 085 245 B1**
(45) Date of publication and mention of the grant of the patent: **13.03.2024**
(21) Application number: 20839018.7
(22) Date of filing: 21.12.2020
(51) Int. Cl.: G01N 21/64, G01N 21/27, G01N 33/542, G01N 33/72, G01N 33/68

(54) **METHOD AND DEVICE FOR ANALYSIS OF LIQUID SAMPLES**
VERFAHREN UND VORRICHTUNG ZUR ANALYSE VON FLÜSSIGKEITSPROBEN
PROCÉDÉ ET DISPOSITIF D'ANALYSE D'ÉCHANTILLONS LIQUIDES

(30) Priority: 31.12.2019 DK PA201901565
(43) Date of publication of application: 09.11.2022
(73) Proprietor: Qlife ApS, 2100 Copenhagen (DK)
(72) Inventor: WARTHOE, Peter, 2100 Copenhagen Ø (DK); FINDING, Ebbe, 2820 Gentofte (DK); ELKJÆR, Robert, 3460 Birkerød (DK)
(74) Representative: COPA Copenhagen Patents
(86) International application number: PCT/EP2020/087510
(87) International publication number: WO 2021/136715

(56) References cited:
- JP-A- 2014 169 946
- US-A1- 2010 137 120
- US-A1- 2016 103 089

## Description

### Technical Field

The present invention relates to methods and a device for quantitatively detecting the presence or absence of an analyte in a liquid sample with improved sensitivity, precision and total assay time. In particular, the present invention relates to a method and a device for quantitatively detecting the presence or absence of a biomarker in a blood sample. Further, the invention relates to a method and a device for detecting the presence or absence of a viral specimen, in particular detection of covid-19, in a liquid sample with improved sensitivity, precision and total assay time.

### Background

Many diseases may be monitored by monitoring the presence or absence of a particular analytes, such as markers or biomarkers, in body fluid samples, particularly by monitoring the presence or absence of particular analytes in blood samples.

When monitoring such disease markers, the sensitivity, precision, and total assay time (TAT) of the apparatus and methods used for analysis are always important issues.

In a point-of-care apparatus and a method for analysing a liquid, such as blood, it is not practically acceptable to use sample sizes of more than 50 µl. Often, the sample size is restricted to the amount of liquid being present in 1 - 2 drop(s) of blood, i.e. approx. 40µl or 20 µl, or less. When working with such small volumes of samples, the sensitivity, precision and TAT of the apparatus and the methods used for analysis become highly significant issues, and ways of increasing the sensitivity, precision and/or TAT of the apparatus and methods are always challenging.

Previously, when analysing a small amount of a liquid sample, such as analysis of one drop of blood, improvements in sensitivity, precision and TAT have been achieved in a range of different ways.

Some methods and apparatuses focus on providing improvements with respect to accurately metering a small volume sample, whereby increased precision may be obtained. Recently, a new approach, volumetric absorptive microsampling, permits the collection of a fixed volume of whole blood.. The technique involves the use of a sampler with a porous hydrophilic tip that enables the collection of small, accurate and precise blood volumes. The collection process usually takes around 2-4 seconds regardless of the haematocrit level. After drying, the samples can be stored, transported, or directly analysed. The technique is gaining more and more attention because of its simplicity and cost effectiveness. The purpose of the technique is to improve test reliability by providing a fixed volume sample of blood and facilitate self-sampling with minimal instructions.

Others focus on providing improved methods and apparatuses capable of accurately mixing the constituents of a particular sample and the detection means (fluorophores, transmission, absorbance etc.), whereby increased sensitivity and precision may be obtained.

Other methods focus on improving the quality of the sample material (e.g. by removing undesired constituents of the sample) used for the analysis. However, accurate analysis of specific analytes present in a liquid sample represents a ubiquitous problem in the art, especially for methods and apparatuses for point-of-care home applications.

Accordingly, one object of the present invention is to improve the sensitivity, precision and TAT of existing devices and methods based on optical measurements that are capable of quantitatively detecting the presence or absence of one or more analytes in a liquid sample, such as a liquid sample comprising less than 50 µL.

Optically based methods are to be understood as methods relying on an optical measuring system where a source of electromagnetic radiation irradiates a liquid sample present in a container (e.g. a cuvette), whereafter the absorption and/or emission of electromagnetic radiation from the sample in the container (e.g. cuvette) is monitored.

Further, since the emergence of the COVID-19 pandemic, a need for rapid nucleic-acid-based tests that can be performed by non-professionals outside the laboratory has arisen. Such tests could help the containment of the pandemic SARS-CoV-2 virus and prevent further lockdown of cities and countries.

Accordingly, another object of the present invention is to provide a sensitive, precise, and rapid nucleic-acid-based tests of the presence or absence of covid-19 infectious agents in a sample.

In general, methods wherein an optical measuring system monitors the amount of transmission and/or absorption of irradiation by a sample present in a container (e.g. cuvette) in a detection apparatus are well-known in the art. Conventionally, a series of measurements are performed (including a blank measurement) in between which specific reagents are introduced to the sample reagent mixture. After each introduction of sample and/or reagent, the materials are mixed thoroughly by various techniques, most often by performing oscillations (vortexing) of the container comprising the material.

### Brief description of the invention

The present invention relates to an improved method and device capable of quantitatively detecting the presence or absence of one or more analytes in a liquid sample, such as a liquid sample comprising less than 50 µL based on optical measurements.

An important modification of the general assay procedures, according to the present invention, is that after receipt of sample and assay reagents into the container (part of the "detection assembly" of the detection apparatus), the entire optical measuring system, i.e. the detection assembly comprising the optical measuring system and the container comprising the sample, is submitted to oscillating motions ("vortexed"), as opposed to conventional oscillations of only the means for receiving the liquid (the container). In other words, the entire optical system, including the assay capsule/cuvette and the source of radiation and the detector, is performing oscillating movements for mixing the constituents of the detection liquid. Thereby, the precision and accuracy of the assay were seen to be significantly improved. This modification is embodied in the Egoo device optical unit used below in the examples. The technical benefits of the invention appear from the examples below.

Accordingly, in one highly preferred aspect, the invention relates to a method for measuring the amount of an analyte in a sample, the method comprising the steps of:
a. providing a detection assembly, said detection assembly comprising
   i. a source of electromagnetic radiation,
   ii. a means for detecting electromagnetic radiation and
   iii. a container containing a sample-free liquid, said container being positioned between the source of electromagnetic radiation and the means for detecting electromagnetic radiation such that applied electromagnetic radiation from the source may radiate through the container from the source to the detection means, or alternatively such that applied electromagnetic radiation from the source may result in the emission of electromagnetic radiation from the liquid in the container to the detection means,
b. adding a sample to the sample-free liquid in the container,
c. optionally adding one or more reagents,
d. providing a measurement of electromagnetic radiation at time T₀,
e. optionally adding one or more reagents,
f. providing a measurement of electromagnetic radiation at time T₁,
g. optionally providing one or more additional sample measurements of electromagnetic radiation,

wherein steps b-d may be performed in any order,
the method being characterised in that the sample and the liquid in the container are subjected to at least one mixing step (m) performed by oscillating the detection assembly in a circular or ellipse motion by vortexing, whereby the positioning of the container relative to the source and the means is constant during said mixing step.

### Definitions

### Precision

The precision of an analytical procedure expresses the closeness of agreement (degree of scatter) between a series of measurements obtained from multiple sampling of the homogeneous sample under the prescribed conditions. Precision may be considered at three levels: 1) repeatability, 2) intermediate precision, and 3) reproducibility. Repeatability expresses the precision under the same operating conditions over a short interval of time. Repeatability is also termed intra-assay precision. Intermediate Precision expresses within-laboratories variations: different days, different analysts, different equipment, etc. Reproducibility expresses the precision between laboratories (collaborative studies usually applied to standardization of methodology). Precision should be investigated using homogeneous, authentic (full scale) samples. However, if it is not possible to obtain a full-scale sample it may be investigated using a pilot-scale or bench-top scale sample or sample solution. The precision of an analytical procedure is usually expressed as the variance, standard deviation or coefficient of variation of a series of measurements.

### Accuracy

Accuracy is a description of systematic errors, a measure of statistical bias, as these cause a difference between a result and a "true" value. In simplest terms, given a set of data points from repeated measurements of the same quantity, the set can be said to be precise if the values are close to each other, while the set can be said to be accurate if their average is close to the true value of the quantity being measured. The concepts of accuracy and precision are independent of each other, so a particular set of data can be said to be either accurate or precise, both accurate and precise, or neither accurate nor precise.

### Optical detection methods

Any optical detection method may ultimately be used to detect the presence of analytes in samples according to the inventions described herein. These include spectroscopic and spectrophotometric methods of analysis. The use of spectrophotometers spans various scientific fields, such as physics, materials science, chemistry, biochemistry, and molecular biology.

Spectroscopy and spectrophotometry are conventionally used for quantitative measurement of the absorption, reflection and/or transmission properties of a material (an analyte) as a function of wavelength of light absorbed/emitted from the sample. The use of these techniques is well known in the art.

Absorption spectroscopy refers to spectroscopic techniques that measure the absorption of radiation, as a function of frequency or wavelength, due to its interaction with a sample. The sample absorbs energy, i.e., photons, from the radiating field. The intensity of the absorption varies as a function of frequency, and this variation is the absorption spectrum. Absorption spectroscopy is performed across the electromagnetic spectrum.

Absorbance spectroscopy, commonly referred to as spectrophotometry, is the analytical technique based on measuring the amount of light absorbed by a sample at a given wavelength. Spectrophotometry, particularly in the visible and UV portions of the electromagnetic spectrum, is one of the most versatile and widely used techniques in chemistry and the life sciences. Absorption spectroscopy is employed as an analytical chemistry tool to determine the presence of a particular substance in a sample and, in many cases, to quantify the amount of the substance present. Infrared and ultraviolet-visible spectroscopy are particularly common in analytical applications. Absorption spectroscopy is also employed in studies of molecular and atomic physics, astronomical spectroscopy, and remote sensing.

There is a wide range of experimental approaches for measuring absorption spectra. The most common arrangement is to direct a generated beam of radiation at a sample and detect the intensity of the radiation that passes through it. The transmitted energy can be used to calculate the absorption. The source, sample arrangement and detection technique vary significantly depending on the frequency range and the purpose of the experiment.

Fluorescence spectrometry is a fast, simple, and inexpensive method to determine the concentration of an analyte in a solution based on fluorescent properties. It can be used for relatively simple analyses, where the type of compound to be analyzed (the analyte) is known, e.g. to perform a quantitative analysis to determine the concentration of the analyte in the samples. Fluorescence is used mainly for measuring compounds in a solution.

In fluorescence spectroscopy, an electromagnetic beam passes through a solution in a cuvette, and an analyte in the sample absorbs energy from the beam. This energy is emitted as an electromagnetic beam (light) with a different wavelength. The amount of light that is absorbed and emitted by the sample is proportional to the presence of analyte in the sample. In fluorescence spectrometry, both the excitation spectrum (the light that is absorbed by the analyte) and/or an emission spectrum (the light emitted by the exited analyte) can be measured. The concentration of the analyte is directly proportional with the intensity of the emission.

Turbidimetry is the process of measuring the loss of intensity of transmitted light due to the scattering effect of particles suspended in it. Light is passed through a filter creating a light of known wavelength which is then passed through a cuvette containing an assay solution. A photoelectric detector collects the light which passes through the cuvette. A measurement is then given for the amount of absorbed light.

Immunoturbidimetry is an important tool in the broad diagnostic field of clinical chemistry. It is used to determine proteins not detectable with classical clinical chemistry methods. Immunoturbidimetry uses the classical antigen-antibody reaction. The antigen-antibody complexes are particles which can be optically detected by a photometer. In more detail, liquid sample is added to a buffer solution and mixed with a suspension of monoclonal antibody against analyte that is bound to latex. The analyte binds to the latex-bound antibody and agglutinates. The light scattering caused by the increase in particle size is used as a measure of analyte concentration. The amount of light scattering is proportional to the concentration of analyte in the sample.

### General procedures when performing optical detection methods

Conventionally, optical detection methods rely on the introduction of a liquid sample directly into a container (e.g. a "cuvette") and the measurement of the change in an optical signal generated by the presence of the sample. Normally, the container contains a sample-free liquid prior to the introduction of the sample. The reaction liquid may contain certain reagents that may interact with the analyte in the sample to produce a signal in the presence of the analyte. Alternatively, such reagents are added after introduction of the sample. The container may e.g. in certain methods contain a fluorophore, which upon the arrival of a liquid in the container may be solubilised.

A sample blank measurement may be performed to provide a background reference. The background measurement is performed in order to correct the sample measurements for unspecific signal ("noise"), which is signal generated by other constituents than the analyte in the detection liquid as well as the influence of the system (e.g. the container) on the signal. Unspecific signals could be generated, e.g. by blood haemolysis, effecting the quality of filtrated plasma/serum. Thus, the generalised methods contain steps of providing a sample blank measurement by measuring the transmission and/or emission of electromagnetic radiation at one or more wavelengths through the first liquid at time T₀.

In this respect, the T₀ measurements is measured prior to the introduction of the sample or, alternatively, prior to (or immediately following) introducing the reagent providing a quantitative change in the transmission/emission of radiation from the sample and providing the signal generated by the background in the sample. Repeated measurements may be performed in order to increase the precision of the blank measurement.

The introduced sample/reagent alters the transmission or emission of electromagnetic radiation at one or more wavelengths through the detection liquid, and the degree of alteration reflects the degree of presence of the analyte in the introduced sample. In other words, the introduction of sample/reagent produces an alteration in a detectable radiation-based signal, and the alteration is quantitatively proportional to the amount of sample present (e.g. determined by use of internal standards with known concentration of sample).

After introducing the sample to the reagents, the constituents of the generated detection liquid must be mixed thoroughly in order to generate an accurate and precise sample measurement.

In a conventional laboratory setup, the person performing the method will often use the metering device (e.g. a pipette) which is repeatedly emptied and reloaded in the detection liquid in the cuvette in order to secure the accurate emptying of sample constituents into the detection liquid and, at the same time, the proper mixing of the components of the detection liquid prior to analysis.

In automated systems, the repeated emptying and reloading of the metering device by sucking and releasing the liquid back and forth from the metering device to the detection liquid has proven problematic. In point-of-care apparatuses and methods, it is often difficult, if not impossible, to apply repeated introduction and reuptake of detection liquid in the means introducing the sample into the first detection liquid, as such apparatuses and methods generally operate in automated and closed compartments in order to minimise exposure to the external environment.

Instead, in conventional automated systems, a metering device empties the sample into the detection liquid, which is then mixed by subjecting the container (cuvette) to rapid oscillations in a circular motion, i.e. vortexing the cuvette containing the detection liquid prior to the performance of the actual sample measurement.

After mixing, a sample analyte measurement is performed conventionally by measuring the transmission and/or emission of electromagnetic radiation at one or more wavelengths through the detection liquid at time T₁. Repeated measurements may be performed in order to increase the precision of the measurement. Thus, the time T₁ reflects one or more measurements performed after introducing the sample and mixing the detection liquid.

### Detailed disclosure of the invention

During the experiments leading to the present invention, the general approach described above proved to be less sensitive, accurate and less precise than desired.

Surprisingly, the inventors of the present invention found that the conventional approach could be improved significantly in terms of sensitivity and precision by not only oscillating the container containing the detection liquid, but by mixing of the contents of the detection liquid by oscillating the entire detection assembly, i.e. including the source of electromagnetic radiation, the means for detecting electromagnetic radiation and the container containing the detection liquid.

The reason for the improved sensitivity and precision is not clear. However, in the conventional automated analysis, the sample constituents are mixed prior to analysis by vortexing the cuvette containing the detection liquid, either in the assembly device or outside the assembly device. The consequence of this procedure is that the cuvette is not positioned in the exact same position with respect to the source and the detection means in the blank and the sample measurement.

Surprisingly, securing the positioning of the cuvette in the exact same location in both measurements provided remarkable improvements in the sensitivity and precision in the measurements.

It is speculated that minor differences and impurities in the container material may give rise to these differences, which compromise the sensitivity and precision of the conventional measurements.

The positioning of the container (cuvette) in the exact same location in both the sample blank measurements and the sample measurement was surprisingly obtained according to the present invention by subjecting the source of the electromagnetic radiation and the means for detecting electromagnetic radiation to the same movements as the container and the sample by vortexing the entire detection assembly.

Thereby, the positioning of the container relative to the source of electromagnetic radiation and the means for detecting electromagnetic radiation is constant throughout the completion of the method steps from the blank measurement at time T₀ to the sample measurements at time T₁.

Further, the potential contamination of the external surfaces of the container containing the liquid sample is minimised, due to less handling of the container. The method of the present invention is therefore highly suited for point-of-care applications at home.

Thus, in one preferred aspect, the present invention relates to a method for measuring the amount of an analyte in a liquid sample, the method comprising the steps of:
a. providing a detection assembly, said detection assembly comprising
   i. a source of electromagnetic radiation,
   ii. a means for detecting electromagnetic radiation and
   iii. a container containing a sample-free liquid, said container being positioned between the source of electromagnetic radiation and the means for detecting electromagnetic radiation such that applied electromagnetic radiation from the source may radiate through the container from the source to the detection means, or alternatively such that applied electromagnetic radiation from the source may result in the emission of electromagnetic radiation from the liquid in the container to the detection means,
b. adding a sample to the sample-free liquid in the container,
c. optionally adding one or more reagents,
d. providing a measurement of electromagnetic radiation at time T₀,
e. optionally adding one or more reagents,
f. providing a measurement of electromagnetic radiation at time T₁,
g. optionally providing one or more additional sample measurements of electromagnetic radiation,

wherein steps b-d may be performed in any order,
the method being characterised in that the sample and the liquid in the container are subjected to at least one mixing step (m) performed by oscillating the detection assembly in a circular or ellipse motion by vortexing, whereby the positioning of the container relative to the source of electromagnetic radiation and the means for detecting electromagnetic radiation is constant during said mixing step.

The analyte detection assembly comprises means for receiving a container (said means and container being positioned between the source and the detection means such that electromagnetic radiation radiates from the source through the container and the liquid to the detection means).

In one embodiment, a mixing step (m) is performed subsequently to step b and prior to step d.

In one embodiment, a mixing step (m) is performed subsequently to step d and prior to step f.

In one preferred embodiment a mixing step (m) is performed subsequently to step f.

The invention also comprises a device for performing the above methods, said device comprising:
a. a detection assembly comprising:
   i. a source of electromagnetic radiation,
   ii. means for detecting electromagnetic radiation, and
   iii. a container comprising a sample-free liquid or a means for receiving a container comprising a sample-free liquid,
b. means for providing oscillations in a circular or ellipse motion of the detection assembly by vortexing.

It should be noted that the skilled person is aware of how to provide means for providing oscillations in a circular or ellipse motion of the detection assembly. Such means are basically conventional means for providing oscillating motions of a particular matter, combined with fastening the parts of the detection assembly to each other, such that all parts of the detection assembly are subjected to the provided motions.

The present disclosure also comprises a kit-of-parts for performing the above methods, said kit-of-parts comprising:
A) a container containing a sample-free liquid, and
B) a device comprising:
   a. a detection assembly comprising
      i. a source of electromagnetic radiation,
      ii. means for detecting electromagnetic radiation, and
      iii. means for receiving a container comprising a sample-free liquid, and
   b. means for providing oscillations in a circular or ellipse motion of the detection assembly by vortexing.

Preferably, the container A) also comprises a compartment comprising a filter material for collection of a metered amount of sample.

Preferably, the container A) also comprises compartments comprising one or more reagents for performing a particular reaction with a particular analyte.
the present disclosure also relates to an apparatus capable of performing the method according to the invention. In one embodiment, the invention relates to the Egoo device as described in more detail below.

### Egoo device:

The Egoo device is a Micro Opto Electro Mechanical device capable of performing the present invention. The entire Egoo device consists of an optical unit and an Egoo capsule for the measurement of biomarkers in human blood or another suitable detection liquid. The disposable assay capsules contain all assay reagents for performing an assay. The assay capsules are inserted into Egoo device, and the assay is thereafter run automatedly.

Essentially, the Egoo device consists of a detection assembly consisting of a source of light and a detector situated such that an assay cuvette can be placed in between the source of light and the detector. The Egoo device further comprises means for vortexing the entire detection assembly when an Egoo capsule is added to the assembly. The Egoo device is supplemented with a capsule comprising an assay cuvette and a separate chamber comprising a filter material on which liquid sample may be added.

Thus, the Egoo device is a kit-of-parts designed for point-of-care use by unexperienced users.

The kit of parts comprises a measuring device and a capsule for receiving sample material.

### Egoo optical unit:

The Egoo optical unit comprises a conventional optical measuring system comprising a detection assembly comprising a source of electromagnetic radiation, a means for detecting electromagnetic radiation and a means for receiving a container ("cuvette") comprising a liquid, said means and container being positioned between the source and the detection means such that electromagnetic radiation radiates through the liquid from the source to the detection means. After receipt of the container comprising a sample liquid, the device is capable of subjecting the entire optical measuring system (the detection assembly) to oscillating motions ("vortexing"), as opposed to conventional devices and methods wherein oscillations of only the container comprising the liquid are performed.

The optical system located inside the detection assembly consists generally of two optical paths. In the examples 1-3 below, the transmission in optical path 1 is measured at 570nm using the LED570nm as light source and the photodiode 1 as detector measuring the absorbance signal. In the examples 1-3 below, in optical path 2, the light source is the LED390 and the photodiode 1 is the detector for measuring the fluorescence signal. PKU measurements are made using the optical path 2, whereas haemoglobin is measured using optical path 1.

In example 4, the transmission in optical path 1 is measured at 460nm/530nm using a LED460nm as light source and the photodiode 1 as detector, measuring the fluorescence signal at 530nm (Cybr-green).

### Egoo capsule

The Egoo capsule comprises a main cuvette (sometimes referred to as the reaction chamber) as well as separate compartments. More precisely, the Egoo capsule consists of a sample injector compartment R1, fluid chambers R2, R3 and main cuvette R4. The Egoo device may add the constituents of R1, R2 and/or R3 to the main cuvette (R4) depending on the relevant assay.

Further, the capsule contains plungers/seal breakers which can be activated such that reagents or material from each compartment may come into fluid communication with the liquid in the main cuvette after breakage of a liquid-impermeable seal and/or enter the main cuvette by being injected down through the sealing into the cuvette.

### Measurement of COVID-19 in samples

As a response to the rapidly increasing demand for point-of-care COVID-19 tests, the present inventors also surprisingly found that the well-known SIBA amplification technique (PLoS One. 2014 Nov 24;9(11):e112656. doi: 10.1371/journal.pone.0112656. eCollection 2014) could be used to rapidly detect COVID-19 directly from swap samples from patients in the Egoo device without the need for performing prior nucleic acid extraction from the samples.

Isothermal nucleic acid amplification technologies, such as the well-known "Strand Invasion Based Amplification" (SIBA), offer significant advantages over polymerase chain reaction (PCR) in that they do not require thermal cycling or sophisticated laboratory equipment.

The SIBA technology relies on the recombinase-dependent insertion of an invasion oligonucleotide (IO) into the double-stranded target nucleic acid. The duplex regions peripheral to the IO insertion site dissociate, thereby enabling target-specific primers to bind. A polymerase then extends the primers onto the target nucleic acid leading to exponential amplification of the target. The primers are not substrates for the recombinase and are therefore unable to extend the target template in the absence of the IO. The inclusion of 29-O-methyl RNA to the IO ensures that it is not extendible and that it does not take part in the extension of the target template. These characteristics ensure that the technology is resistant to nonspecific amplification since primer dimers or mis-priming are unable to exponentially amplify. Consequently, SIBA is highly specific and able to distinguish closely related species with single molecule sensitivity in the absence of complex probes or sophisticated laboratory equipment.

During the investigations leading to the present invention, it was surprisingly found that application of swap samples from patients tested for infection with coronavirus (COVID-19) using the SIBA amplification technique did not require extraction of nucleic acid from the sample. Accordingly, the conventional swap samples (samples containing the swap from the patient dissolved in approximately 1 ml buffer solution) could be tested efficiently and the SIBA amplification technique was efficient in positively determining patients having the COVID-19 infection. However, the protocol was found to give a significant number of false positive test results. Surprisingly, dilution of swap samples in buffer (various) solution solved this problem without negatively affecting the efficacy in determining true positive samples. Thus, it was surprisingly found that by diluting the sample in a suitable buffer, it was possible to minimize or avoid false positive test results. Specifically, it was found that diluting swap samples in a total final volume of 2 ml minimized false positives. Better results were obtained by diluting samples in a final volume of 5 ml buffer/swap. However, in order to totally avoid unspecific reactions (false positives), dilution of the sample was required to a final volume of more than 5 ml/swap, such as 10 ml/swap. Accordingly, it was found that the swap samples (standard swap samples consisting of a swap head diluted in 1-2 ml buffer) diluted in 10 times volume of buffer (volume/volume) avoided any false positive results without giving rise to an increase in false negative samples. In practice, the swap samples (around 1 ml sample material) should therefore be diluted in 10 ml buffer before being added to the reaction mixture in the Egoo device, resulting in an almost total avoidance of false positives. However, in order to minimize the number of false positives, a lesser degree of dilution could suffice.

Accordingly, the present disclosure also relates to a method for detecting the presence of COVID-19 in a sample, the method comprising the steps of:
a. providing a swap sample from a patient,
b. diluting the swap sample in a dilution buffer in a volume of at least 2 ml, preferably at least 3 ml, more preferably at least 4 ml, even more preferably at least 5 ml, even more preferably at least 6 ml, even more preferably at least 7 ml, even more preferably at least 8 ml, even more preferably at least 9 ml, even more preferably at least 10 ml dilution buffer,
c. subjecting a subsample of the diluted sample to a reaction mixture suitable for performing SIBA amplification,
d. subjecting the sample and the reaction mixture to a reaction temperature suitable for the applied SIBA amplification,
e. detecting the presence or absence of a reaction product, wherein the presence of a reaction product is interpreted as a detection of presence of COVID-19 in the sample, and the absence as a detection of absence of COVID-19,
f. optionally, subjecting the samples, wherein the presence of COVID-19 in the sample is detected, to a melting curve analysis for verifying the positive detection.

Preferably, the dilution buffer is a buffer containing mild detergents such as the detergent listed below. Preferably the dilution buffer is a buffer suitable for SIBA amplification, such as the SIBA buffer described in the examples below.

The SIBA buffer used in the examples consisted of the following reagents: 33.17 ml nuclease-free water, 3.2 ml 1 M Mg-acetate, 0.8 ml Tris-acetate, pH 8, 2.67 ml 30% Brij-58, 0.12 ml 10% ASB-14, and 0.04 ml 100% Proclin 300 (total 40 ml), giving a final concentration in the buffer of 80 mM Mg-acetate, 20 mM Tris-acetate pH 8, 2% Brij-14, 0.03% ASB-14 and 0.1% Proclin.

The COVID-19 amplicon detected in the assay had the following sequence: GAACTTTAAGTCAGTT**CTT**TATTATCAAAACAATGTTTTTATGTCTGAAGCAAAA**TGTT**GGACTGAGACTG

The strand-invasive probe had the following sequence: CCCCCCCCCCCCC**CTT**TATTATCAAAACAATGTTTTTATGTCTGAAGCAAAA**TGTT**.

The two primers targets had the following sequences:
GAACTTTAAGTCAGTT**CTT** (primer 1)
**TGTT**GGACTGAGACTG (primer 2)

Other preferred embodiments of this invention appear from example 4 below.

### Preferred embodiments of invention

### Sample material

Preferably, the liquid sample is a sample consisting of less than 40 µl of liquid. Such sample size is relevant for automated methods and apparatuses. More preferably, the liquid sample is a sample consisting of less than 20 µl of liquid. Such sample size is relevant for point-of-care apparatuses and methods.

In a highly preferred embodiment, the sample to be analysed is a blood sample. In one preferred embodiment, the blood sample is whole blood. In another preferred embodiment, the blood sample is a blood plasma sample.

### Mixing

In the above-mentioned methods, it is highly preferred that the mixing of the contents of the detection liquid is performed by rapidly oscillating the detection liquid ion in a circular of ellipse motion at a speed of at least 1000 rpm. Preferably, the mixing is performed by rapidly (1000-4000 rpm) oscillating the detection liquid in a circular of ellipse motion (vortexing).

### Source of electromagnetic radiation

The detection assembly comprises a source of electromagnetic radiation, a source of electromagnetic radiation being defined as a means from which electromagnetic radiation is emitted. The relevant electromagnetic radiation may in principle be of any suitable wavelength. However, electromagnetic radiation in the wavelength between 300nm and 900nm is preferred.

### Means for detecting electromagnetic radiation

The analyte detection assembly comprises a means for detecting electromagnetic radiation, a means for detecting electromagnetic radiation being defined as a means with which electromagnetic radiation is detected (i.e. absorbed and converted into electrical energy). The relevant electromagnetic radiation to be detected may in principle be of any suitable wavelength. However, the electromagnetic radiation to be detected must be suitable in view of the electromagnetic radiation being emitted by the source and/or by the sample.

### Analytes

In general, the methods and apparatuses of the invention can be used to measure all blood biomarkers within clinical chemistry, cancer diagnostics and all other related diagnostics fields.

The methods and apparatuses of the inventions are, however, preferably used for the detection of one or more of the following blood markers (analytes); Phenylalanine (phenylketonuria patients), CRP, hs-CRP, Lipid Panel (inflammation and cardiovascular vascular disease biomarkers), Lipid profile (total cholesterol, HDL and Triglyceride), HbA1c (diabetes biomarkers), ALAT (liver biomarker), Vitamin D and D-dimer.

In a preferred embodiment of the invention, the reaction liquid comprises a substance which binds to an analyte present in the sample, such as the fluorophore eosin-borate-acid for HbA1 detection.

### EXAMPLES

The purpose of the examples below is to describe and compare the assay precision, %CV (standard deviation / mean * 100) using the described invention.

### Example 1. PKU measurement

Phenylketonuria (PKU) is autosomal recessive genetic disorder caused by a deficiency of hepatic phenylalanine hydroxylase (PAH) activity. In the Caucasian population, about 1 in 50 are carriers, and 1 in 10.000 are affected with PKU. Because of the PAH deficiency, phenylalanine is not converted to the amino acid tyrosine. This causes an excessive amount of PHE and toxic metabolites to accumulate in all parts of the body, including the brain, in blood, and in urine. Those excesses create a chemical imbalance that results in various degrees of mental retardation. During the last decade, several vendors have tested various assay methods to identify the best method for measuring the PHE levels at home, methods that must be comparable to measuring blood sugar at home for people with diabetes. Unfortunately, the phenylalanine molecule is present in 500-1000x lower concentration in blood (µM) compared to glucose (mM). So far, all attempts to identify home-based methods have been struggling with assay-related parameters, such as assay sensitivity, assay precision, assay stability, assay comparison and assay complexity. The present invention has succeeded in overcoming the above assay-related challenges.

Using the described invention, it is possible to perform such PHE assay in the small Egoo POC device in the users' home having identical or even better analytical performance compared to the state-of-the-art laboratory-based equipment.

### PHE Assay principle

The Phenylalanine assay makes use of a fluorescence ninhydrin assay method. The assay procedure is a modification of the fluorometric assay procedure first published by McCaman and Robin, Lab Clin. Med 59, page 885-890 in 1962. The assay is based on a chemical method intended for the quantitative determination of PHE in blood.

### Summary of the PHE assay procedure according to the invention

A precise volume of capillary blood (15 µl) is transferred from a finger to a blood-metering transfer pipette comprising a capillary channel capable of containing 15 ml sample. The blood-metering transfer pipette is inserted into the capsule inlet of an Egoo capsule, where it is brought into contact with a membrane material (Whatman-903). When the metering transfer pipette gets into physical contact with the membrane material, the blood is passively flowing from the capillary channel in the pipette into the membrane material. After a drying period, the membrane comprising the sample is injected into the main cuvette where the amino acid phenylalanine (and all other amino acids) is extracted out of the membrane by use of an extracting solution (R1) and an oscillating (vortex) movement of the detection assembly comprising the cuvette inside the Egoo device. Next, the R2 reagent is injected into the main cuvette and mixed according to the invention. After incubating at 48 °C (45-80 °C), the PHE now forms a fluorescence compound with ninhydrin. The fluorometric response and specificity is greatly enhanced by the presence of a dipeptide L-leucyl-L-alanine. The pH during the reaction is strictly controlled by a succinate buffer at 5.8+/- 0.1 to maximize specificity. After the ninhydrin reaction, the pH is adjusted to >8.0 for optimal fluorescence detection by injecting the R3 solution into the main cuvette. The fluorescence molecule is measured at 450nm with the excitation wavelength being 390nm.

### PHE Assay reagents:

R1: sample injector containing the membrane
R2: 70mM Ninhydrin in water.
R3: 0.3M Na2HPO4, 0.05M NaOH, pH=11.5.
R4: 70% Ethanol, 0.2M Succinate buffer pH 4.9, 0.4% NaCl, 10mM L-leucyl-L-alanine.

### PKU procedure:

1. 15µl of full blood is added to a blood-metering transfer pipette,
2. The metering transfer pipette is inserted into the inlet of the Egoo PHE capsule,
3. When the metering transfer pipette gets into physical contact with the Whatman903 membrane material in R1, the blood is passively flowing into the membrane material. After the blood transfer, the metering transfer pipette is discarded,
4. After blood transfer to the Whatman-903 membrane, the filter is dried for 3 hours.
5. After drying, the blood membrane from R1 is injected into the main cuvette where the extracting solution R4 combined with an oscillating (vortex) movement are extracting (releasing) PHE molecules (and other amino acids) into the extracting solution R4,
6. The R2 reagent is injected into the main cuvette, and the fluorescence assay is initiated,
7. A sample blank measurement by measuring the fluorescence to time T₀ is taken,
8. The assay mixture is incubated for 30-90 minutes at 48 °C,
9. The R3 reagent is injected into the main cuvette to adjust PH to >8.0 for fluorescence enhancement,
10. Next *T₁,* fluorescence measurement is taken, and the result is calculated *T₁* / *T₀,*
11. Finally, the PHE concentration is calculated by using a calibration curve to translate the raw fluorescence data to a final PHE concentration.

### Results

The following analytical performance characteristics tests are determined:
Precision
- Intra precision study (intravariability)
- Inter precision study (intervariability)

Blood samples containing approx. 50µM and 500µM phenylalanine (blood sample 1 and blood sample 2) are assayed.

*Intra-precision.* The variation experienced by a single operator on a single device within a single series of PHE measurements.

**Table 1. Intra-precision study for the PHE assay. Total of PHE 60 runs at the two concentrations using one Egoo device n= 2 × (2 × 15) = 60 runs.**

| | **PKU assay; n=15** | |
|---|---|---|
| **Egoo-9** | **Mean dose** | **CV %** |
| **PHE blood sample 1** | ∼50 µM (sample 1) | 9.3 |
| **PHE blood sample 2** | ∼500 µM (sample 2) | 5.1 |

*Inter-precision study.* Inter precision is variation within a laboratory, between days, different instruments, and different operators. The results are shown in Table 2 below.

**Table 2. Inter-precision.**

| | **PKU assay; n=15** | |
|---|---|---|
| **Egoo-9 & 3** | **Mean dose** | **CV %** |
| **PHE blood sample 1** | ∼50 µM (sample 1) | 9.9 |
| **PHE blood sample 2** | ∼500 µM (sample 2) | 6.7 |

### Discussion

The purpose of the example is to explore the possibility of integrating a well-known fluorescence-based PHE assay together with the present invention. The result indicates that the method according to the invention shows excellent performance.

### Example 2. Haemoglobin assay

### Haemoglobin Assay principle

Haemoglobin is a routine diagnostic parameter.

In this embodiment of the invention, the well-known SLS haemoglobin detection method using cyanide-free sodium lauryl sulphate (SLS) is used. The reagent lyses red and white blood cells in the sample. The chemical reaction begins by altering the globin and then oxidising the haeme group. Thereafter, the SLS' hydrophilic groups can bind to the haeme group and form a stable, coloured complex (SLS-HGB), which is analysed using a photometric method.

In the Egoo device, a LED (570nm) sends out monochromatic light which is absorbed by the SLS-HGB complexes when travelling through the mixture. The absorbance is measured by a photo sensor and is proportional to the haemoglobin concentration of the sample.

### Summary of the Hb assay procedure

A precise volume of capillary blood (15 µl) is transferred from a fingertip to a blood-metering transfer pipette. The blood-metering transfer pipette is inserted into the capsule inlet, and the sample is transferred to a filter material by passive transfer. The sample containing filter material is injected/inserted into the main cuvette, where the blood is instantly extracted out of the membrane using vortex movement of the detection assembly. The Hb now forms SLS-HGB complexes that can be measured at 570nm after 2 minutes incubation with the R1 reagent.

### Haemoglobin Assay reagent

R4: Commercially available SLS haemoglobin detection reagent (Sysmex).

After metering and adding the blood to the assay capsule, all assay steps was performed by the Egoo device:
1. 15µl of full blood from a finger stick is added to a blood-metering transfer pipette,
2. The metering transfer pipette is inserted into the inlet of the Egoo Hb capsule, where the metering transfer pipette gets into physical contact with a membrane material. After the blood transfer, the metering transfer pipette is discarded,
3. A sample blank measurement by measuring the absorbance to time T₀ is taken,
4. After blood transfer to the membrane on R1, the blood is injected into the main cuvette, where the blood is mixed with the R4 reagent using an oscillating (vortex) movement of the detection assembly,
5. After 2 minutes, a *T₁*, absorbance measurement is taken, and the result is calculated *T₁* / *T₀,*
6. Finally, Hb concentration is calculated by using a calibration curve to translate the raw absorbance data to a final Hb concentration.

### Analytical performance characteristics

Intra-precision. The intra-precision is the variability experienced by a single operator on a single device within a single series of haemoglobin measurements.

### Results

Results are shown in Table 3 below. Intra-precision study for the Haemoglobin assay.

10 Hb assays are run at two Hb concentration.

A total of 2 × 10 = 80 Hb assay runs on Egoo.

**Table 3. Repeatability of haemoglobin assay.**

| **Precision %CV** | |
|---|---|
| **Mean dose** | **Haemoglobin assay** |
| 4.0 mM | 1.9 |
| 10.0 mM | 2.4 |

### Discussion:

The purpose of example 2 is to explore the possibility of integrating a well-known absorbance-based Hb assay together with the present invention.

### Example 3

The purpose of this example is to compare the precision (%CV) using the present invention wherein reagent/sample mixing is performed by vortexing the detection assembly, including the assay cuvette, the source of light and the detector, with a similar method wherein reagent/sample mixing is performed by vortexing the assay cuvette outside the detection assembly.

50 µl of a liquid comprising the fluorescence dye EosinY (excitation 370nm and emission 550nm) is added to fluid chamber R2. 50 µl of a liquid comprising the dye bromophenol blue (max absorbance 570nm) is added to fluid chamber R3. 300 µl of a liquid consisting of a phosphate solution is added to the main cuvette R4. The capsule is inserted into the Egoo device. The Egoo measuring procedure is described below.

### Egoo measuring procedure 1 (procedure according to the invention):

1. The capsule is inserted into the Egoo device,
2. A blank signal from the main cuvette (phosphate buffer) is measured,
3. The R2 solution is injected into the main cuvette and mixed by a vortex movement according to the invention (5 seconds, 1500 rpm) followed by measuring the fluorescence and absorbance signal (signal 1 and signal 2),
4. The R3 solution is injected into the main cuvette and mixed by a vortex movement according to the invention (5 seconds, 1500 rpm) followed by measuring the fluorescence and absorbance signal (signal 3 and signal 4),
5. The capsule is taken out of the Egoo device and discarded,
6. The procedure is repeated 20 times using 20 capsules (n=20).

### Procedure 2 (reference procedure wherein all mixing steps of the reagents are performed outside the Egoo device, and all measurements steps are performed inside the Egoo device)

50 µl of a liquid consisting of the fluorescence dye EosinY (excitation 370nm and emission 550nm) is added to fluid chamber R2. 50 µl of a liquid consisting of the dye bromophenol blue (max absorbance 570nm) is added to fluid chamber R3. 300 µl of a liquid consisting of a phosphate solution is added to the main cuvette R4. The capsule is inserted into Egoo measuring according to the procedure described below.

### Egoo procedure 2:

1. The capsule is inserted into the Egoo device,
2. A blank signal from the main cuvette (phosphate buffer) is measured,
3. The R2 solution is injected into the main cuvette,
4. The capsule is removed from the Egoo device, and a manual mixing step is performed by using a vortex mixing (5 seconds, 1500 rpm),
5. The capsule is again inserted into the Egoo device followed by measuring the fluorescence and absorbance signal,
6. The R3 solution is injected into the main cuvette,
7. The capsule is removed from the Egoo device, and a manual mixing step is performed using a vortex mixing (5 seconds, 1500 rpm),
8. The capsule is again inserted into the Egoo device followed by measuring the fluorescence and absorbance signal,
9. The capsule is taken out of the Egoo device and discarded,
10.The procedure is repeated 20 times using 20 capsules (n=20).

### Assay reagents

R2: Eosin Y 0,5 w/v solution, Sigma Aldrich reagent 2853,
Lot 01838
R3: Bromophenol Blue solution, Sigma Aldrich reagent 313744,
lot MKCD9662
R4: Standard phosphate buffer, Ph 7.4

**Table 4. Precision %CV, n=20 (20 capsules used for each of the two procedures)**

| | Procedure 1 | Procedure 2 |
|---|---|---|
| %CV at signal 1 (fluorescence) | 0.5 | 8.6 |
| %CV at signal 2 (absorbance) | 1.1 | 8.1 |
| %CV at signal 3 (fluorescence) | 0.4 | 7.9 |
| %CV at signal 4 (absorbance) | 1.3 | 7.3 |

### Discussion:

The purpose of the example is to compare the precision (%CV) using the present invention with a similar method not using the present invention.

As can be observed from Table 4 above, the fluorescence and absorbance signal precision using the present invention is significantly improved. The major reason for this improvement in precision has probably to do with the better alignment between the main cuvette (assay unit) and the optical system when the mixing and measuring step are performed as described in the present invention.

### Example 4. Detection of SARS-CoV-2 using Reverse Transcription Strand Invasion Based Amplification in the Egoo device.

In the present example, the Egoo monitoring system is used for extraction-free detection of SARS-CoV-2 using isothermal Reverse Transcription Strand Invasion Based Amplification (RT-SIBA).

The SARS-CoV-2 RT-SIBA assay is performed directly on crude oropharyngeal and nasopharyngeal swabs without nucleic acid extraction and results in a detectable reaction within a time of 30 minutes.

The Egoo device and capsule are ideal for this type of analysis, as the Egoo capsule system is automatically sealed tight in the Egoo device after applying the sample, resulting in a closed system optimal for molecular isothermal amplification. As seen below, the Egoo device is as sensitive and specific as a PCR instrument with an analytical sensitivity of 25 viral RNA copies per reaction for the SARS-CoV-2 RT-SIBA assay and a clinical sensitivity and specificity of 93% and 100% respectively.

### Sample preparation

Oropharyngeal or nasopharyngeal swabs are collected with flocked swabs (Jiangsu Hanheng Medical Technology, Copan) and dissolved directly into 1ml SIBA lysis/reaction buffer, 1 ml PBS, 1-4 ml Saline, 1 ml UTM (Copan), 1 ml VTM (NEST Biotechnology) or 1ml VTM (Mole Bioscience). Samples dissolved in PBS, Saline, UTM or VTM are 10-fold diluted in SIBA lysis/reaction buffer (80 mM Mg-acetate, 20 mM Tris-acetate (pH 8), 2% Brij-58, 0.03% ASB-14, 0.1% Proclin 300) before being applied to the SARS-CoV-2 RT-SIBA mastermix (Qlife). Viral RNA from the Nasopharyngeal swabs is also purified using the QiaAmp Viral RNA kit (Qiagen) according to the manufactures instructions before being analyzed with the SARS-CoV-2 RT-PCR assay.

### SARS-CoV-2 RT-SIBA

The SARS-CoV-2 RT-SIBA mastermix (Qlife) consists of mixing 3 reagents to be mixed according to the manufacturer's (Aidian) instructions. Thaw the RT-SIBA Mix A (reaction mix containing enzymes for recombination and amplification), Mix B (reaction mix containing co-factors) and oligomix (reaction mix containing primers and invasive probe) on ice and mix by vortexing. Details of the mastermix reagents can be found by contacting the manufacturer (Aidian) or by consulting e.g. "Hoser MJ, Mansukoski HK, Morrical SW, Eboigbodin KE. Strand Invasion Based Amplification (SIBAH): A Novel Isothermal DNA Amplification Technology Demonstrating High Specificity and Sensitivity for a Single Molecule of Target Analyte. PLOS ONE | DOI:10.1371/journal.pone.0112656 November 24, 2014" or "Eboigbodin K, Filén S, Ojalehto T, Brummer M, Elf S, Pousi K, Hoser M. Reverse transcription strand invasion-based amplification (RT-SIBA): a method for rapid detection of influenza A and B. Appl Microbiol Biotechnol (2016) 100:5559-5567DOI 10.1007/s00253-016-7491-y". The primers and the invasive probe in the oligomix were as stated herein.

In some instances, Mix A can form precipitates, which can be removed by heating to 37-41°C followed by vortexing. For reference reactions performed in a PCR instrument, the mastermix is prepared by mixing 7 µl Mix A, 7 µl Mix B, and 3.5 µl Oligomix per reaction. Mastermix (17.5 µl) is added to a PCR tube, and 2,5 µl of sample (diluted 10-fold in SIBA lysis/reaction buffer) is added to the mastermix. The RT-SIBA reactions are performed using either the MX3005P (Strategene) or CFX96 (BioRAD), and setting the PCR instrument to 44 °C and recording fluorescence measurements every minute for 30 minutes, followed by a melt curve analysis: 44 °C-95 °C. For reactions performed in the Egoo device, Egoo capsules (Qlife) containing 140 µl of premade mastermix are loaded into the Egoo device after applying 20 µl of the sample (10-fold diluted in SIBA lysis/reaction buffer). Before the reaction starts, the Egoo capsule is closed in the Egoo device once the piston mechanism seals the capsule with the plunger. Once the plunger has sealed the capsule tight, the Egoo device heats the capsule to 44 °C for 30 minutes. During the reaction, the reagents within the capsule are mixed by shaking (1000 rpm) for 3 seconds every 5 minutes. Fluorescence measurements are recorded 3 times every minute.

### SARS-CoV-2 RT-PCR (reference)

The E-gene assay from Charit6 Berlin is used with the Luna^{®} Universal Probe One-Step RT-qPCR Kit (NEB). Briefly described, 12,5µl Luna Universal One-Step Reaction Mix, 1,25µl Luna WarmStart^{®} RT Enzyme Mix, 0,5µl E_Sarbeco_F1 (20 µM), 0,5µl E_Sarbeco_R2 (20 µM), 0,25µl E_Sarbeco_P1 (20 µM), 7,5µl nuclease-free water and 2,5µl sample (purified RNA or sample diluted 10-fold in SIBA lysis/reaction buffer) are mixed and run with the following program: 10 min. at 55°C, 3 min. at 95°C, 45 cycles of 15 sec. at 95°C and 30 sec. at 58°C. The RT-PCR reactions are performed using either the MX3005P (Strategene), CFX96 (BioRAD), or AriaMx (Agilent).

### Virus culture

Inactivated virus cultures for Epstein-Barr Virus (EBV)(B95-8), Parainfluenza virus type 1 (PIV-1), Adenovirus type 5 (Adv5), Respiratory Syncytial virus type A (RSV-A)(2006), Influenza A (H1N1)pdm (INFL A)(NY/02/07), Influenza A (H3N2), Influenza B (INFL B)(Yamagata/16/88), Rhinovirus A16, Enterovirus type 68 (EV-68)(2007), Human metapneumovirus (hMPV) (Peru2-2002), Coronavirus OC43, Coronavirs NL63, Coronavirus 229E, SARS-COV-2 (Italy-INMI1)(1.02 × 108 TCID50/mL), SARS-COV-2 (USA-WA1/2020)(3.09 × 108 TCID50/ml), SARS-COV-2 (Hong Kong/VM2000i06i/2020)(1.15 × 107 TCID50/mL) purchased from Helvetica Health Care are used directly by spiking into an oropharyngeal swab background resulting in a 10-fold dilution of the virus. QCMD panels for MERS (2019), RSV (2019), hMPV (2019) and coronavirus (2019) are purified using the MagNA Pure 96 system (Roche) and the DNA and Viral NA Small Volume Kit (Roche). The human 2019-nCoV isolate (026V-03883)(EVAg) was cultured in Vero E6 cells, and the virus titre of the supernatant is determined to 1.6 × 105 TCID50/ml. In addition, the harvested supernatant is quantified to 1.2 × 107 RNA copies/ml using MagNA Pure purified RNA and a standard curve based on the synthetic SARS-CoV-2 RNA control (Twist Bioscience) spiked into RNA from an oropharyngeal swab. The quantification is performed using the RT-PCR E-gene assay.

### Clinical samples

Twenty-five positive nasopharyngeal swabs (Central BioHyb^{®}), 25 negative nasopharyngeal swabs (Central BioHyb^{®}), 30 positive oropharyngeal swabs (Qlife COVID-19 Service Center), and 30 negative oropharyngeal swabs (Qlife COVID-19 Service Center) are tested for SARS-CoV-2 using the RT-SIBA assay and the RT-PCR assay. Patient-informed consent is obtained for each sample tested.

The developed isothermal SARS-CoV-2 RT-SIBA assay target the RdRp gene in SARS-CoV-2.

RT-SIBA amplicon:

GAACTTTAAGTCAGTTCTTTATTATCAAAACAATGTTTTTATGTCTGAAGCAAAATGTTGGACTGAGACTG
CCCCCCCCCCCCCCTTTATTATCAAAACAATGTTTTTATGTCTGAAGCAAAATGTT
GAACTTTAAGTCAGTTCTT TGTTGGACTGAGACTG

### Results

Silico analysis of full-length sequences from 119,215 viral sequences submitted to GISAID shows that 99.62% of all isolates worldwide would be detected by the SARS-CoV-2 RT-SIBA assay primers and probe as described above (452 sequences worldwide contained non-unique random SNPs).

For methodological simplification, the SARS-CoV-2 RT-SIBA assay is designed to be used directly on crude samples without NA extraction using a SIBA lysis/reaction buffer, which contains mild detergents and magnesium.

First, the analytical specificity of the SARS-CoV-2 RT-SIBA assay against other human coronaviruses (hCoV-NL63, hCoV-229E and hCoV-OC43) and the most common human respiratory viruses such as Influenza A H1N1, Influenza B and Respiratory Syncitial virus (RSV) is confirmed. Purified viral NA or inactivated virus cultures from the different viruses are spiked into a SARS-CoV-2 negative oropharyngeal background.

The samples were diluted 10-fold in SIBA lysis/reaction buffer before being analyzed with the SARS-CoV-2 RT-SIBA assay using two different PCR instruments (MX3005P or CFX96) and the small Egoo device. No cross-reactivity to other respiratory viruses is detected for either the purified NA or whole lysed virus cultures (non-purified RNA), and the SARS-CoV-2 RT-SIBA assay was 100% specific for SARS-CoV-2. Thereafter, the analytical sensitivity of the SARS-CoV-2 RT-SIBA assay is investigated. Synthetic SARS-CoV-2 RNA and inactivated SARS-CoV-2 virus culture is spiked into negative oropharyngeal swab at different concentrations and diluted 10-fold in SIBA lysis/reaction buffer. The different dilutions are tested using both a PCR instrument (Mx3005P) and five different Egoo devices.

### Analytical sensitivity of the SARS-CoV-2 assay

**Table 5. na; not analyzed, no; number, Pos; positive, min; minutes, SD; standard deviation, the bold highlights define the limit of detection**

| **Sam-ple material** | | **SARS-CoV-2 RT-SIBA in PCR instrument¹** | | | | **SARS-CoV-2 RT-SIBA in Egoo device** | | |
|---|---|---|---|---|---|---|---|---|
| | **Viral load (cp/µl)** | **Copies to RT-SIBA reaction (cp)** | **No. of Pos. (%)** | **Min. to pos. (mean ± SD)** | **Viral load (cp/µl)** | **Copies to RT-SIBA reac-tion** | **No. of Pos. (%)** | **Min. to pos. (mean ± SD)** |
| **Synthetic RNA²** | 1.0×10⁵ | 2.5×10⁵ | 6/6 (100) | 8.5 ± 0.5 | 1.0×10⁵ | 2.0×10⁶ | 8/8 (100) | 16.0 ± 1.5 |
| | 1.0×10⁴ | 2.5×10⁴ | 6/6 (100) | 9.6 ± 0.1 | 1.0×10⁴ | 2.0×10⁵ | 6/6 (100) | 16.7 ± 2.1 |
| | 1.0×10³ | 2.5×10³ | 6/6 (100) | 10.3 ± 0.4 | 1.0×10³ | 2.0×10⁴ | 6/6 (100) | 17.2 ± 1.7 |
| | 1.0×10² | 2.5×10² | 6/6 (100) | 11.7 ± 0.5 | 1.0×10² | 2.0×10³ | 6/6 (100) | 19.8 ± 2.3 |
| | **1.0×10¹** | **2.5×10¹** | **6/6 (100)** | **14.3 ± 1.1** | 1.0×10¹ | 2.0×10² | 6/6 (100) | 21.5 ± 3.3 |
| | 1.0×10⁰ | 2.5×10⁰ | 5/6 (83) | 16.9 ± 0.9 | **1.0×10⁰** | **2.0×10¹** | **7/7 (100)** | **25.6 ± 3.3** |
| | 1.0×10⁻¹ | 2.5×10¹ | na | na | 1.0×10⁻¹ | 2.0×10⁰ | 1/8 (13) | 22.9 |
| | 0 | 0 | 0/6 | 0 | 0 | 0 | 0/6 | 0 |
| **Virus culture³** | 1.2×10³ | 2.9×10³ | 64/64 (100) | 12.6 ± 1.1 | 1.2×10³ | 2.4×10³ | 2/2 (100) | 16.3 ± 0.7 |
| | 1.2×10² | 2.9×10² | 64/64 (100) | 15.9 ± 1.9 | 1.2×10² | 2.4×10² | 1/1 (100) | 21.88 |
| | 1.0×10¹ | 2.6×10¹ | 33/33 (100) | 20.8 ± 3.0 | 1.2×10¹ | 1.0×10² | 2/2 (100) | 21.4 ± 0.7 |
| | **9.3×10⁰** | **2.3×10¹** | **32/33 (97)** | **19.9 ± 2.2** | 6×10⁰ | 5.0×10¹ | 2/2 (100) | 21.9 ± 1.4 |
| | **8.1×10⁰** | **2.0×10¹** | **33/33 (100)** | **20.1 ± 1.8** | **1.3×10⁰** | **2.5×10¹** | **19/20 (95)** | **21.8** ± **1.8** |
| | 6.3×10⁰ | 1.6×10¹ | 31/33 (94) | 21.9 ± 3.9 | 1×10⁰ | 2.0×10¹ | 9/20 (45) | 23.9 ± 2.9 |
| | 0 | 0/33 | 0 | 0 | 0 | 0/6 | | 0 |

| | | | | | | | | |
|---|---|---|---|---|---|---|---|---|
| ¹ MX3005P ² Twistsyntetic SARS-COV-2 RNA ctrl ³ 2019-nCoV isolate (026V-03883)(EVAg) | | | | | | | | |

The limit of detection (LOD) of the SARS-CoV-2 RT-SIBA assay is found to be between 20-25 RNA copies/reaction when using both the synthetic RNA and whole virus culture. No difference between the LOD is observed between the PCR instrument and the five Egoo devices tested. However, for the PCR instrument only 2.5 µl of sample is loaded into the PCR tube, whereas 20 µl of sample is loaded into the Egoo capsule. This corresponds to a lower sample input concentration for the Egoo instrument compared to the PCR instrument. The Egoo device could detect as low as 1.25 viral RNA copies/µl.

The SARS-CoV-2 RT-SIBA assay is designed to be used directly on crude samples without NA extraction using a SIBA lysis/reaction buffer. To test if lysis can be performed directly in the sample collection tube, different concentrations of SARS-CoV-2 virus culture are spiked into a negative oropharyngeal swab background and subsequently added to sampling swabs (n=16). SIBA lysis/reaction buffer (500µl) is added directly to the tubes containing the spiked swabs and the tubes are incubated for 10 min at RT with shaking to release the viral RNA from the swab, before being analysed by the SARS-CoV-2 RT-SIBA assay using a PCR instrument. As negative controls, negative oropharyngeal swabs (n=18) directly dissolved SIBA lysis/reaction buffer are also analysed. Swabs spiked with 4200 virus cp and dissolved in SIBA lysis/reaction buffer to a concentration of 79 virus cp/µl could easily be detected with the SARS-CoV-2 RT-SIBA reaction. Analysis of SARS-CoV-2 negative oropharyngeal swabs shows positive detection in 39% (7/18) of the samples, and analysis of the melting curve shows a second melting peak around 56°C, whereas the correct melting peak for a SARS-CoV-2 positive sample should be at 68°C. The SARS-CoV-2 assay is based on SYBR Green detection, and therefore non-target amplification can occur due to the genomic background in the sample. Non-target amplification is easily detected in a PCR instrument by performing melt curve analysis. However, melt curve analysis cannot be performed in the Egoo device. It is therefore investigated if dilution of the samples by 2-, 5-, 10-, 20-fold would reduce non-target amplification. Dilution of the samples clearly shows that 10- and 20-fold dilutions of the samples eliminate the formation of the non-target melting peak. To further test this, 128 negative oropharyngeal patient swabs (dissolved in 1ml PBS) are 10-fold diluted in SIBA lysis/reaction buffer and analysed with the SARS-CoV-2 RT-SIBA assay. Only 1/128 (0.7%) of the 10-fold diluted negative oropharyngeal swabs shows non-target amplification, with a very small amplification curve. These results show that samples should preferably be diluted 10-fold in SIBA lysis/reaction buffer before being analysed on the Egoo device. To methodologically simplify the dilution procedure for non-professionals, 10 ml of SIBA lysis/reaction buffer must be added to a sample collection tube if the sample is to be used directly in the SARS-CoV-2 RT-SIBA assay on the Egoo device.

Since samples should preferably be diluted 10-fold to reduce non-target amplification in the oropharyngeal swab, we wanted to investigate whether another sampling media such as UTM and VTM can be used with the Egoo monitoring system. Since we only have access to SARS-CoV-2 positive oropharyngeal patient samples dissolved in PBS or Saline, we simulate SARS-CoV-2 positive UTM and VTM samples by spiking virus culture (Hong Kong/VM2000i06i/2020) or SARS-CoV-2 positive oropharyngeal patient samples (originally dissolved in 1 ml PBS) in UTM (Copan), VTM (NEST Biotechnology), and VTM (Mole Bioscience) (1:10). The simulated UTM and VTM samples are further diluted 10-fold in SIBA lysis/reaction buffer before the samples are analysed with the SARS-CoV-2 RT-SIBA assay. The SARS-CoV-2 RT-SIBA assay is not influenced by the presence of UTM (Copan), VTM (NEST Biotechnology), or VTM (Mole Bioscience), and all dilutions of the virus culture and all patient samples are detected except the SARS-CoV-2 negative swab which remains negative. No significant difference between the different media is observed. These results show that the SARS-CoV-2 RT-SIBA assay is not dependent on the sample collection method, and samples already collected in different VTM and UTM media can be used in the SARS-CoV-2 RT-SIBA assay.

The direct lysis and 10-fold dilution of the sample could potentially result in a lower clinical sensitivity of the SARS-CoV-2 RT-SIBA assay. To test this, the clinical sensitivity is tested on 30 SARS-CoV-2 positive and 30 SARS-CoV-2 negative oropharyngeal patient swabs dissolved in 1ml PBS previously diagnosed with RT-PCR for the E-gene (Qlife COVID-19 Service Center). Additional 25 SARS-CoV-2 positive and 25 SARS-CoV-2 negative nasopharyngeal patient swabs dissolved in 1-4 ml saline and previously diagnosed with Hologic Panther SARS-CoV-2, FilmArray Respiratory panel 2 and Perkin Elmer SARS-CoV-2 purchased from Central BioHub are also analyzed. The collected samples are diluted 10-fold in SIBA lysis/reaction buffer and tested on the Egoo instrument. These results show a clinical sensitivity and specificity of 93% and 100% for the oropharyngeal swabs in PBS.

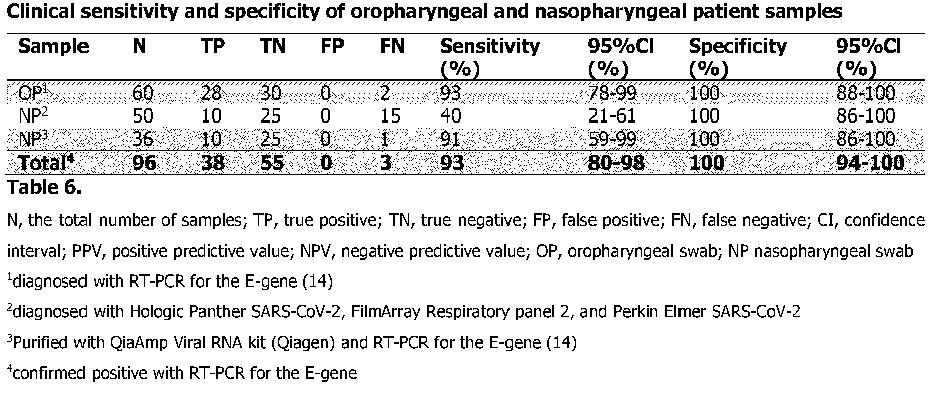

Surprisingly, only 10/25 (40%) of the nasopharyngeal swabs in saline are detected (Table 6 above). The nasopharyngeal swabs are purchased from Central BioHub, where the samples have been stored at -80 degrees for an unknown number of days before shipment from abroad. We therefore decided to confirm the presence of SARS-CoV-2 in the samples. RNA from the nasopharyngeal swabs are purified using the QiaAmp Viral RNA kit (Qiagen) and then analysed for SARS-CoV-2 RNA using the E-gene assay (14). Only 11/25 (44%) of the samples could be confirmed positive by RT-PCR (Table 6) and only 1/11 of these samples are not detected by the SARS-CoV-2 RT-SIBA assay (Table 6). Removing these degraded samples from the calculations, the total sensitivity and specificity of the SARS-CoV-2 RT-SIBA assay in nasopharyngeal and oropharyngeal swabs are 93% and 100%, respectively (Table 6).

### Discussion

Rapid nucleic-acid based tests performed by non-professionals outside the laboratory can help in the containment of the COVID-19 pandemic. So far, only the less sensitive antigen tests and one nucleic-acid based test have been developed and commercialised for non-professional use (https://www.lucirahealth.com/). Here, we present for the first time a new and very small instrument called the Egoo monitoring system, which has been developed for home-use monitoring of biochemical markers. The Egoo monitoring system is simple to use and can be used in private homes, primary care clinics, nursing homes, mass transport hubs (airports or train stations) and workplaces without the need for specialized laboratory staff.

For methodological simplicity, we have developed an extraction-free SARS-CoV-2 RT-SIBA assay that uses a specialized SIBA lysis/reaction buffer containing mild detergents. During the COVID-19 pandemic, NA extraction has proved not only to be time-consuming, but also to cause a bottleneck due to lack of consumables. Therefore, many laboratories have been forced to look for alternative methods to NA extraction, such as direct use of the crude sample using either heat or detergents for inactivation and lysis of the virus. This has proven to be almost as sensitive and specific as the gold standard purification methods, and after optimizing, we ended with a simplified sampling workflow for the SARS-CoV-2 RT-SIBA assay that can be used by non-professionals using the Egoo instrument.

One disadvantage of the simplified workflow is that samples must be diluted in a buffer (such as the SIBA lysis/reaction buffer) to be specific in the Egoo device. If performing the SARS-CoV-2 assay in a PCR instrument, a melting curve analysis can be performed to test the specificity of the assay. However, this is not possible in the Egoo device and therefore sample dilution must be performed, e.g. by adding a higher sampling volume to the collection tube. A high sampling volume will most likely influence the sensitivity of the assay, and samples containing a very low viral load might not be suitable for the Egoo device. Other studies have shown that direct use of nasopharyngeal samples dissolved in PBS, Saline, and UTM without NA extraction inhibits direct RT-PCR and dilution of samples (or reducing the sample input volume into the RT-PCR reaction) reduce the inhibitory effect. The 10-fold dilution of the sample before analysis in the Egoo device will therefore not only eliminate non-target amplification but may also eliminate inhibitors that might otherwise influence the SARS-CoV-2 RT-SIBA reaction.

The Egoo instrument uses specialized capsules that are sealed tight with a plunger within the Egoo device after applying the sample, and thus eliminates the risk of amplicon contamination. The amplification steps in nucleic acid-based test are extreme, resulting in billions of copies of the target of interest. This amplification step requires a closed system to avoid amplicon contamination and the detection of false positives. Opening a tube after an amplification to use it on a lateral flow stick is possible to do in a specialised laboratory but is not possible to do outside a laboratory in the current form without contaminating the entire surroundings. An alternative to the lateral flow stick is the LuciraTM COVID-19 All-in-One single-use Test kit that has recently received FDA authorization (https://www.lucirahealth.com/). The all-in-one single-use eliminates the need for opening the tube after amplification. In contrast to the Lucira single use test kit, the Egoo device can be used endless times and only requires replacing the assay capsule after use. Therefore, the Egoo device can be used for many subsequent assays, including tests for other respiratory viruses.

## Claims

1. A method for measuring the amount of an analyte in a sample, the method comprising the steps of:
a. providing a detection assembly, said detection assembly comprising:
i. a source of electromagnetic radiation,
ii. a means for detecting electromagnetic radiation, and
iii. a container containing a sample-free liquid, said container being positioned between the source of electromagnetic radiation and the means for detecting electromagnetic radiation such that applied electromagnetic radiation from the source may radiate through the container from the source to the detection means, or alternatively such that applied electromagnetic radiation from the source may result in the emission of electromagnetic radiation from the liquid in the container to the detection means,
b. adding a sample to the sample-free liquid in the container,
c. optionally adding one or more reagents,
d. providing a measurement of electromagnetic radiation at time T₀,
e. optionally adding one or more reagents,
f. providing a measurement of electromagnetic radiation at time T₁,
g. optionally providing one or more further sample measurements of electromagnetic radiation,
wherein steps b - d may be performed in any order,
the method being **characterised in that** the sample and the liquid in the container are subjected to at least one mixing step (m) performed by oscillating the detection assembly in a circular or ellipse motion by vortexing, whereby the positioning of the container relative to the source and the means is constant during said mixing step.

2. The method according to claim 1, wherein a mixing step (m) is performed subsequently to step b and prior to step d.

3. The method according to any one of claims 1-2, wherein a mixing step (m) is performed subsequently to step d and prior to step f.

4. The method according to any one of claims 1-3, wherein a mixing step (m) is performed subsequently to step f.

5. The method according to any one of claims 1-4, wherein the sample is a liquid sample consisting of less than 50 µl.

6. The method according to any one of claims 1-5, wherein the mixing in step (m) is performed by oscillating the detection assembly in a circular or ellipse motion at a speed of at least 1000 rpm by vortexing.

7. The method according to any one of claims 1-6, where the sample is a blood sample.

8. The method according to any one of claims 1-7, where the analyte is selected from the group comprising phenylalanine, hs-CRP, HbA1c, vitamin D, d-dimer or a blood lipid.

9. Device for performing the method according to claims 1-8, said device comprising:
a. a detection assembly comprising:
i. a source of electromagnetic radiation,
ii. means for detecting electromagnetic radiation, and
iii. a container comprising a sample-free liquid or a means for receiving a container comprising a sample-free liquid,
the device being **characterized by**
b. means adapted for providing oscillations in a circular or ellipse motion of the detection assembly by vortexing.

## Patentansprüche

1. Verfahren zum Messen der Menge eines Analyten in einer Probe, wobei das Verfahren die folgenden Schritte umfasst:
a. Bereitstellen einer Erfassungsanordnung, wobei die Erfassungsanordnung folgendes umfasst:
i. eine Quelle elektromagnetischer Strahlung,
ii. ein Mittel zum Erfassen elektromagnetischer Strahlung, und
iii. einen Behälter, der eine probenfreie Flüssigkeit enthält,
wobei der Behälter zwischen der Quelle elektromagnetischer Strahlung und dem Mittel zum Erfassen elektromagnetischer Strahlung positioniert ist, sodass angewandte elektromagnetische Strahlung von der Quelle durch den Behälter von der Quelle zu den Erfassungsmitteln strahlen kann, oder alternativ, sodass angewandte elektromagnetische Strahlung von der Quelle zur Emission von elektromagnetischer Strahlung von der Flüssigkeit in dem Behälter zu den Erfassungsmitteln führen kann,
b. Zugeben einer Probe zu der probenfreien Flüssigkeit in dem Behälter,
c. optional Zugeben eines oder mehrerer Reagenzien,
d. Bereitstellen einer Messung elektromagnetischer Strahlung zum Zeitpunkt T₀,
e. optional Zugeben eines oder mehrerer Reagenzien,
f. Bereitstellen einer Messung elektromagnetischer Strahlung zum Zeitpunkt T₁,
g. optional Bereitstellen einer oder mehrerer weiterer Probenmessungen elektromagnetischer Strahlung, wobei die Schritte b - d in beliebiger Reihenfolge durchgeführt werden können, wobei das Verfahren **dadurch gekennzeichnet ist, dass** die Probe und die Flüssigkeit in dem Behälter mindestens einem Mischschritt (m) unterzogen werden, der durch Oszillieren der Erfassungsanordnung in einer kreisförmigen oder elliptischen Bewegung durch Verwirbeln durchgeführt wird, wobei das Positionieren des Behälters relativ zu der Quelle und dem Mittel während des Mischschrittes konstant ist.

2. Verfahren nach Anspruch 1, wobei ein Mischschritt (m) nachfolgend auf den Schritt b und vor dem Schritt d durchgeführt wird.

3. Verfahren nach einem der Ansprüche 1-2, wobei ein Mischschritt (m) nachfolgend auf den Schritt d und vor dem Schritt f durchgeführt wird.

4. Verfahren nach einem der Ansprüche 1-3, wobei ein Mischschritt (m) nachfolgend auf den Schritt f durchgeführt wird.

5. Verfahren nach einem der Ansprüche 1-4, wobei die Probe eine flüssige Probe ist, die aus weniger als 50 µl besteht.

6. Verfahren nach einem der Ansprüche 1-5, wobei das Mischen in Schritt (m) durch Oszillieren der Erfassungsanordnung in einer kreisförmigen oder elliptischen Bewegung mit einer Geschwindigkeit von mindestens 1000 U/min durch Verwirbeln durchgeführt wird.

7. Verfahren nach einem der Ansprüche 1-6, wobei die Probe eine Blutprobe ist.

8. Verfahren nach einem der Ansprüche 1-7, wobei der Analyt ausgewählt ist aus der Gruppe umfassend Phenylalanin, hs-CRP, HbA1c, Vitamin D, d-Dimer oder ein Blutfett.

9. Vorrichtung zum Durchführen des Verfahrens nach den Ansprüchen 1-8, wobei die Vorrichtung Folgendes umfasst:
a. eine Erfassungsanordnung, umfassend:
i. eine Quelle elektromagnetischer Strahlung,
ii. Mittel zum Erfassen elektromagnetischer Strahlung, und
iii. einen Behälter, der eine probenfreie Flüssigkeit umfasst, oder ein Mittel zum Aufnehmen eines Behälters, der eine probenfreie Flüssigkeit umfasst,
wobei die Vorrichtung **gekennzeichnet ist durch**
b. Mittel, die angepasst sind zum Bereitstellen von Oszillationen in einer kreisförmigen oder elliptischen Bewegung der Erfassungsanordnung durch Verwirbeln.

## Revendications

1. Procédé de mesure de la quantité d'un analyte dans un échantillon, le procédé comprenant les étapes de :
a. fourniture d'un ensemble de détection, ledit ensemble de détection comprenant :
i. une source de rayonnement électromagnétique,
ii. un moyen de détection de rayonnement électromagnétique, et
iii. un récipient contenant un liquide sans échantillon,
ledit récipient étant positionné entre la source de rayonnement électromagnétique et le moyen de détection de rayonnement électromagnétique de sorte qu'un rayonnement électromagnétique appliqué depuis la source puisse rayonner à travers le récipient depuis la source jusqu'au moyen de détection, ou alternativement, de sorte qu'un rayonnement électromagnétique appliqué depuis la source puisse entraîner l'émission d'un rayonnement électromagnétique depuis le liquide dans le récipient jusqu'au moyen de détection,
b. ajout d'un échantillon au liquide sans échantillon dans le récipient,
c. facultativement, ajout d'un ou plusieurs réactifs,
d. fourniture d'une mesure de rayonnement électromagnétique à un temps T₀,
e. facultativement, ajout d'un ou plusieurs réactifs,
f. fourniture d'une mesure de rayonnement électromagnétique à un temps T₁,
g. facultativement, fourniture d'une ou plusieurs mesures d'échantillon de rayonnement électromagnétique additionnelles,
dans lequel les étapes b à d peuvent être mises en oeuvre dans un ordre quelconque,
le procédé étant **caractérisé en ce que** l'échantillon et le liquide dans le récipient sont soumis à au moins une étape de mélange (m) mise en oeuvre par oscillation de l'ensemble de détection dans un mouvement circulaire ou elliptique par agitation au vortex, moyennant quoi le positionnement du récipient par rapport à la source et au moyen est constant pendant ladite étape de mélange.

2. Procédé selon la revendication 1, dans lequel une étape de mélange (m) est mise en oeuvre ultérieurement à l'étape b et antérieurement à l'étape d.

3. Procédé selon l'une quelconque des revendications 1 à 2, dans lequel une étape de mélange (m) est mise en oeuvre ultérieurement à l'étape d et antérieurement à l'étape f.

4. Procédé selon l'une quelconque des revendications 1 à 3, dans lequel une étape de mélange (m) est mise en oeuvre ultérieurement à l'étape f.

5. Procédé selon l'une quelconque des revendications 1 à 4, dans lequel l'échantillon est un échantillon liquide consistant en moins de 50 µl.

6. Procédé selon l'une quelconque des revendications 1 à 5, dans lequel l'étape de mélange (m) est mise en oeuvre par oscillation de l'ensemble de détection dans un mouvement circulaire ou elliptique à une vitesse d'au moins 1000 rpm par agitation au vortex.

7. Procédé selon l'une quelconque des revendications 1 à 6, où l'échantillon est un échantillon sanguin.

8. Procédé selon l'une quelconque des revendications 1 à 7, où l'analyte est choisi dans le groupe comprenant la phénylalanine, le hs-CRP, HbA1c, la vitamine D, un d-dimère ou un lipide sanguin.

9. Dispositif pour mettre en oeuvre le procédé selon les revendications 1 à 8, ledit dispositif comprenant :
a. un ensemble de détection comprenant :
i. une source de rayonnement électromagnétique,
ii. des moyens de détection de rayonnement électromagnétique, et
iii. un récipient comprenant un liquide sans échantillon ou un moyen de réception d'un récipient comprenant un liquide sans échantillon,
le dispositif étant **caractérisé par**
b. des moyens adaptés pour fournir des oscillations dans un mouvement circulaire ou elliptique de l'ensemble de détection par agitation au vortex.
